# EUROPEAN PATENT SPECIFICATION

(11) **EP 4 312 877 B1**
(45) Date of publication and mention of the grant of the patent: **10.06.2026**
(21) Application number: 22716807.7
(22) Date of filing: 23.03.2022
(51) Int. Cl.: A61F 2/00, A61F 2/26, A61F 2/48

(54) **FLUID CONTROL SYSTEM FOR AN IMPLANTABLE INFLATABLE DEVICE**
FLÜSSIGKEITSKONTROLLSYSTEM FÜR EINE IMPLANTIERBARE AUFBLASBARE VORRICHTUNG
SYSTÈME DE COMMANDE DE FLUIDE POUR UN DISPOSITIF GONFLABLE IMPLANTABLE

(30) Priority: 25.03.2021 US 202163200738 P; 22.03.2022 US 202217655952
(43) Date of publication of application: 07.02.2024
(73) Proprietor: Boston Scientific Scimed, Inc., Maple Grove, MN 55311-1566 (US)
(72) Inventor: GILDEA, John, Kildare, W23F40T (IE); SMITH, Noel, Kilkenny, R95 HX88 (IE); MARCOS LARANGEIRA, Eduardo, Clonmel Tipperary, E91 W1R9 (IE); SINNOTT, Thomas, Enniscorthy Wexford (IE); WATSCHKE, Brian P., Minneapolis, Minnesota 55405 (US); NOLAN, Daragh, Waterford, P36 TC98 (IE); BORGOS, Natalie Ann, Roseville, Minnesota 55113 (US)
(74) Representative: Pfenning, Meinig & Partner mbB
(86) International application number: PCT/US2022/071295
(87) International publication number: WO 2022/204699

(56) References cited:
- WO-A1-2014/033062
- DE-A1- 10 239 309
- DE-A1- 102010 032 799
- US-A1- 2009 270 904
- US-A1- 2020 222 188

## Description

### TECHNICAL FIELD

This disclosure relates generally to bodily implants, and more specifically to bodily implants including a pump.

### BACKGROUND

Active implantable fluid operated devices often include one or more pumps that regulate a flow of fluid between different portions of the implantable device. One or more valves can be positioned within fluid passageways of the device to direct and control the flow of fluid so as to achieve inflation, deflation, pressurization, depressurization, activation, deactivation and the like of different fluid filled implant components of the device. In some implantable fluid operated devices, sensors can be used to monitor fluid pressure and/or fluid volume and/or fluid flow within fluid passageways of the device. Accurate monitoring of conditions within the device, including pressure monitoring and flow monitoring, may provide for improved control of device operation, improved diagnostics, and improved efficacy of the device. In addition, sensors could be used to monitor external conditions from the device, including acceleration, angle, barometric pressure and temperature, which facilitate the determination of operating modes of the device.

### BACKGROUND ART

Document US 2020/222188 A1 shows an implantable device, which includes a fluid reservoir configured to be implanted in a body of a patient at a first location, an inflatable member configured to be implanted in the body of the patient at a second location, and a pump assembly configured to be implanted in the body of the patient at a third location. The pump assembly is configured to transfer fluid from the fluid reservoir to the inflatable member in response to the implantable device being in an inflation mode, and the pump assembly configured to transfer the fluid from the inflatable member to the fluid reservoir in response to the implantable device being in a deflation mode. The pump assembly includes an electronic control module, an electronically powered pump, a first valve, and a second valve. The electronic control module is configured to activate or deactivate the electronically powered pump.

Document DE 102 39 309 A1 describes a closure system and a method for selectively opening and closing a tubular body organ, consisting of a closure element and a control system controlling the closure element.

Document US 2009/270904 A1 relates to a method and a system for electronically controlling an artificial fine-sensor sphincter implant, in which method or system at least one sensor signal which behaves analogously to the difference between internal bladder pressure and cuff pressure is converted in an analog or digital electronic circuit by means of computing and comparator functions and compared with reference values in such a way that an actuator is controlled in such a way that the cuff pressure or the differential pressure between cuff and urinary bladder either moves in a low range limited by two threshold values or above a certain safety pressure or, in the case of micturition, below it.

Document DE 10 2010 032799 A1 relates to a microvalve which is formed from two firmly connected substrates and preferably has an actuator element, for example a diaphragm-driven actuator element, for the controlled opening and closing of a first and/or second passage. Furthermore, the documents relates to a manufacturing method for such a microvalve and to a micropump using at least one such microvalve. This micropump is to be used in particular in connection with the development of an artificial sphincter. The microvalve has a first substrate and a second substrate, which are non-detachably connected to each other to form a controllable fluid flow path, and at least one first passage and at least one second passage. The microvalve has at least one elastically deformable sealing structure for sealing the first and/or second passage, which can be formed, for example, by a photostructurable silicone.

Document WO 2014/033062 A1 relates to an artificial sphincter with a hydraulic occlusion device for controllably occluding a urinary vessel in an incontinent patient, characterized by a first electrically driven pump and a second emergency pump. The second emergency pump pumps the hydraulic fluid in the event of improper functioning of the first pump.

### SUMMARY

The invention relates to an implantable fluid operated inflatable device.according to claim 1 and the dependent claims.

According to the present disclosure, an implantable fluid operated inflatable device includes a fluid reservoir; an inflatable member; and a fluid control system configured to transfer fluid between the fluid reservoir and the inflatable member. The fluid control system can include a housing; at least one valve and at least one pump positioned in a fluid passageway within in the housing; a first fluid port in fluidic communication with the fluid reservoir; and a second fluid port in fluidic communication with the inflatable member. The implantable fluid operated inflatable device can also include at least one pressure sensing device configured to sense a fluid pressure in the implantable fluid operated inflatable device; and an electronic control system configured to receive the pressure sensed by the at least one pressure sensing device, and to control the at least one valve and/or at least one pump in response to the received pressure.

In some implementations, the at least one valve and the at least one pump includes a combined pump and valve device positioned inline between the reservoir and the inflatable member, including a chamber; a diaphragm positioned along an edge portion of the chamber; a piezoelectric element mounted on the diaphragm; a first valve positioned at a first end portion of the chamber corresponding to a first end portion of the piezoelectric element; and a second valve positioned at a second end portion of the chamber corresponding to a second end portion of the piezoelectric element. In some implementations, in a first mode in which fluid is moved through the combined pump and valve device in a first direction to transfer fluid from the reservoir to the inflatable member to inflate the inflatable member, a first pumping cycle of the combined pump and valve device can include a first supply stroke in which fluid is drawn into the chamber through the first valve while the second valve is closed; and a first pressure stroke in which fluid is expelled out of the chamber through the second valve while the first valve is closed. In a second mode in which fluid is moved through the combined pump and valve device in a second direction to transfer fluid from the inflatable member to the reservoir to deflate the inflatable member, a second pumping cycle of the combined pump and valve device can include a second supply stroke in which fluid is drawn into the chamber through the second valve while the first valve is closed; and a second pressure stroke in which fluid is expelled out of the chamber through the first valve while the second valve is closed. In some implementations, the first supply stroke and the first pressure stroke are alternately and repeatedly implemented until an inflation pressure of the inflatable member is achieved based on a pressure or other characteristics sensed by the at least one sensing device; and the second supply stroke and the second pressure stroke are alternately and repeatedly implemented until a deflation pressure is achieved based on a pressure sensed by the at least one sensing device.

In some implementations, the at least one valve is a piezoelectric valve, including a valve base or host; at least one inlet port formed in the valve base or host; at least one outlet port formed in the valve base or host; a diaphragm coupled to the valve base or host; and a piezoelectric element mounted on the diaphragm, wherein a voltage applied to the piezoelectric element is a variable voltage to maintain a set state of the fluid operated inflatable device based on a pressure detected in the fluid passageway of the valve relative to a detected pressure external to the valve. The variable voltage applied to the piezoelectric element to maintain the set state of the fluid operated inflatable device may be based on the pressure detected in the fluid passageway of the valve relative to an atmospheric pressure sensed by the electronic control system. The variable voltage applied to the piezoelectric element may adjust a position of the piezoelectric element and the diaphragm so as to adjust at least one of a fluid pressure or a fluid flow rate to adjust for atmospheric conditions and correspond to the set state of the fluid-controlled inflatable device. The voltage applied to the piezoelectric element may be selected from a calibration curve associated with the piezoelectric valve that is accessible in a memory of the electronic control system.

In some implementations, the at least one valve is a normally open piezoelectric valve that is configured to transition from a normally open state to a closed state in response to an application of voltage to the piezoelectric element, and to return to the normally open state in response to release of the voltage. The normally open piezoelectric valve may be configured to remain in the closed state for a period of time after release of the voltage, and to transition to the normally open state in response to dissipation of electrical bias accumulated in the piezoelectric element. In some implementations, the normally open piezoelectric valve includes a resistor electrically connected to the piezoelectric element. The resistor may be configured to control a dissipation of electrical bias accumulated in the piezoelectric element such that the normally open piezoelectric valve transitions from the closed state to the normally open state in a set period of time after release of the voltage.

In some implementations, the piezoelectric valve is a normally closed piezoelectric valve that is configured to transition from a normally closed state to an open state in response to an application of voltage to the piezoelectric element, and to return to the normally closed state in response to release of the voltage. The normally closed piezoelectric valve may include a plunger movably positioned within the fluid passageway of the normally closed piezoelectric valve, wherein the plunger is sealed against the valve base in the normally closed state so as to restrict flow through the fluid passageway, and is spaced apart from the valve base in the open state so as to open the fluid passageway. In the normally closed state of the normally closed piezoelectric valve, a backpressure applied to the plunger through the at least one outlet maintains the sealed position of the plunger against the valve base in response to a surge in fluid pressure at the at least one inlet.

In some implementations, the electronic control system includes a printed circuit board including a memory configured to store at least one control algorithm, a communication module configured to communicate with one or more external devices, and a processor configured to receive pressure level measurements from the at least one sensing device; apply the at least one control algorithm based on the received pressure level measurements; and control operation of the at least one valve and the at least one pump in accordance with the applied at least one control algorithm.

In some implementations, the implantable fluid operated inflatable device is an artificial urinary sphincter or an inflatable penile prosthesis.

In another general aspect, a method of controlling an implantable fluid operated inflatable device includes receiving, by a processor of the inflatable device from a pressure sensing device within a fluid passageway of the inflatable device, a fluid pressure measurement; comparing, by the processor, the measured pressure received from the pressure sensing device to a pressure external to the fluid passageway; and controlling, by the processor, a circuit to apply a voltage to a piezoelectric element of a piezoelectric valve of the inflatable device based on the comparison to maintain a set condition of the inflatable device.

In some implementations, controlling the circuit to apply the voltage to the piezoelectric element includes detecting, based on the comparison, a change in atmospheric pressure from a calibration condition of the inflatable device based on the comparison; selecting, by the processor, a voltage to be applied to a piezoelectric element of a piezoelectric valve of the inflatable device from a previously stored lookup table in response to the detected change in atmospheric pressure; and applying to the selected voltage to the piezoelectric element to maintain a set condition of the inflatable device in the changed atmospheric conditions.

In some implementations, the piezoelectric valve is a normally open piezoelectric valve, and wherein controlling the circuit to apply the voltage to the piezoelectric element includes controlling a resistor in the circuit such that electrical bias accumulated in the piezoelectric element dissipates over a set period of time to return the normally open piezoelectric valve to a normally open state.

In some implementations, the piezoelectric valve is a normally closed piezoelectric valve, and the method also includes detecting a surge in fluid pressure at an inlet portion of the piezoelectric valve; and applying a backpressure at an outlet portion of the piezoelectric valve in response to the surge in fluid pressure at the inlet portion to maintain a closed state of the normally closed piezoelectric valve.

In some implementations, the method also includes receiving, by a control module of the processor, a user input from an external device in communication with the processor; and adjusting at leasta one of a fluid pressure or a fluid flow rate in the inflatable device in response to the received user input.

### BRIEF DESCRIPTION OF THE DRAWINGS

FIG. 1 is a block diagram of an implantable fluid operated device according to an aspect.
FIGS. 2A and 2B illustrate example implantable fluid operated devices according to an aspect.
FIGS. 3A and 3B are schematic diagrams of a fluid architecture of an implantable fluid operated device according to an aspect.
FIGS. 4A and 4B illustrate an open state and a closed state, respectively, of a normally open piezoelectric valve according to an aspect.
FIGS. 5A-5C illustrate a fully open state, a partially open state, and a closed state of a piezoelectric valve according to an aspect.
FIGS. 6A-6C illustrate operation of a normally open valve according to an aspect.
FIG. 7 is a graph of variable closing voltage curves.
FIGS. 8A-8C illustrate operation of an example valve according to an aspect.
FIG. 9 is a schematic diagram of a fluid architecture of an implantable fluid operated device including a valve.
FIGS. 10A-10D illustrate operation of an example pump and valve device according to an aspect.

### DETAILED DESCRIPTION

Detailed implementations are disclosed herein. However, it is understood that the disclosed implementations are merely examples, which may be embodied in various forms. Therefore, specific structural and functional details disclosed herein are not to be interpreted as limiting, but merely as a basis for the claims and as a representative basis for teaching one skilled in the art to variously employ the implementations in virtually any appropriately detailed structure. Further, the terms and phrases used herein are not intended to be limiting, but to provide an understandable description of the present disclosure.

The terms "a" or "an," as used herein, are defined as one or more than one. The term "another," as used herein, is defined as at least a second or more. The terms "including" and/or "having", as used herein, are defined as comprising (i.e., open transition). The term "coupled" or "moveably coupled," as used herein, is defined as connected, although not necessarily directly and mechanically.

In general, the implementations are directed to bodily implants. The term patient or user may hereinafter be used for a person who benefits from the medical device or the methods disclosed in the present disclosure. For example, the patient can be a person whose body is implanted with the medical device or the method disclosed for operating the medical device by the present disclosure.

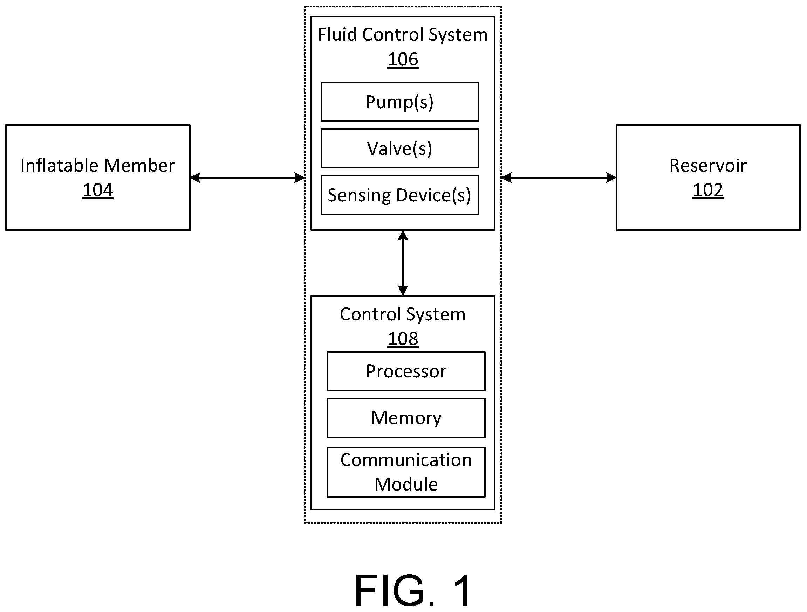

FIG. 1 is a block diagram of an example implantable fluid operated inflatable device 100. The example device 100 shown in FIG. 1 includes a fluid reservoir 102, an inflatable member 104, and a fluid control system 106 including fluidics components such as one or more pumps, one or more valves and the like configured to transfer fluid between the fluid reservoir 102 and the inflatable member 104. The fluid control system 106 can include on or more sensing devices that sense conditions such as, for example, fluid pressure, fluid flow rate and the like within the fluidics system of the device 100. In some implementations, the example device 100 includes an electronic control system 108. The electronic control system 108 may provide for the monitoring and/or control of the operation of various fluidics components of the fluid control system 106 and/or communication with one or more sensing device(s) within the implantable fluid operated inflatable device 100 and/or communication with one or more external device(s). In some examples, the electronic control system 108 includes, for example, a processor, a memory, a communication module, and other such components configured to provide for the operation and control of the implantable fluid operated inflatable device 100. For example, the communication module may provide for communication with one or more external devices. The one or more external devices may be configured to receive user inputs and transmit the user inputs to the electronic control system 108 for processing, operation and control of the device 100. The electronic control system 108 may, through the communication module, transmit operational information to the external device for user consumption. The fluid reservoir 102, the inflatable member 104, and the fluid control system 106 may be internally implanted into the body of the patient. In some implementations, the electronic control system 108 is coupled to or incorporated into a housing of the fluid control system 106. In some implementations, at least a portion of the electronic control system 108 is physically separate from the fluid control system 106. In some implementations, some modules of the electronic control system 108 are coupled to or incorporated into the fluid control system 106, and some modules of the electronic control system 108 are separate from the fluid control system 106. For example, in some implementations, some modules of the electronic control system 108 are included in an external device that is in communication other modules of the electronic control system 108 included within the implantable device 100. In some implementations, operation of the implantable fluid operated inflatable device 100 may be manually controlled.

In some examples, electronic monitoring and control of the fluid operated device 100 may provide for improved patient control of the device, improved patient comfort, and improved patient safety. In some examples, electronic monitoring and control of the fluid operated device 100 may afford the opportunity for tailoring of the operation of the device 100 by the physician without further surgical intervention.

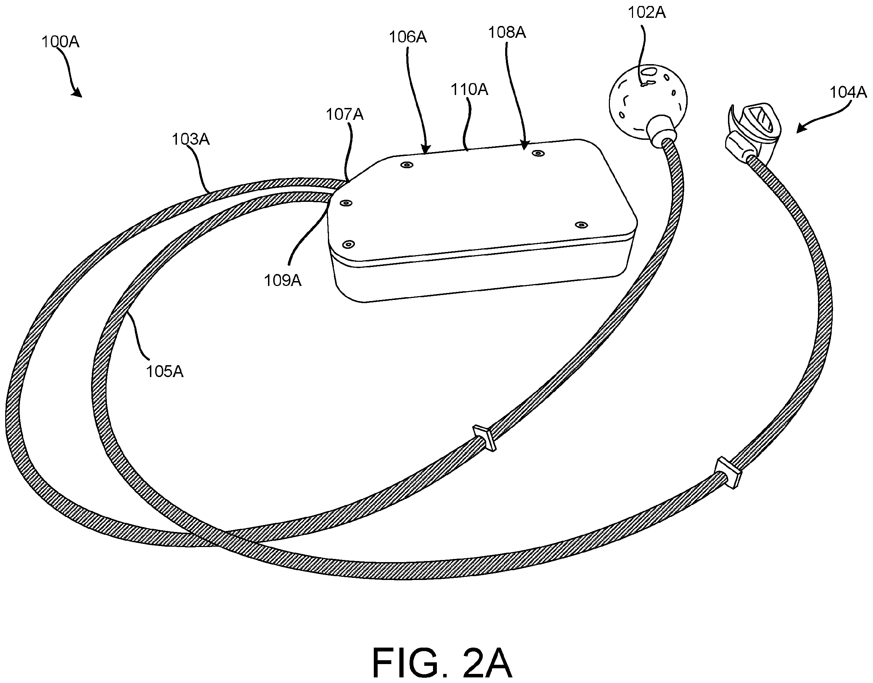

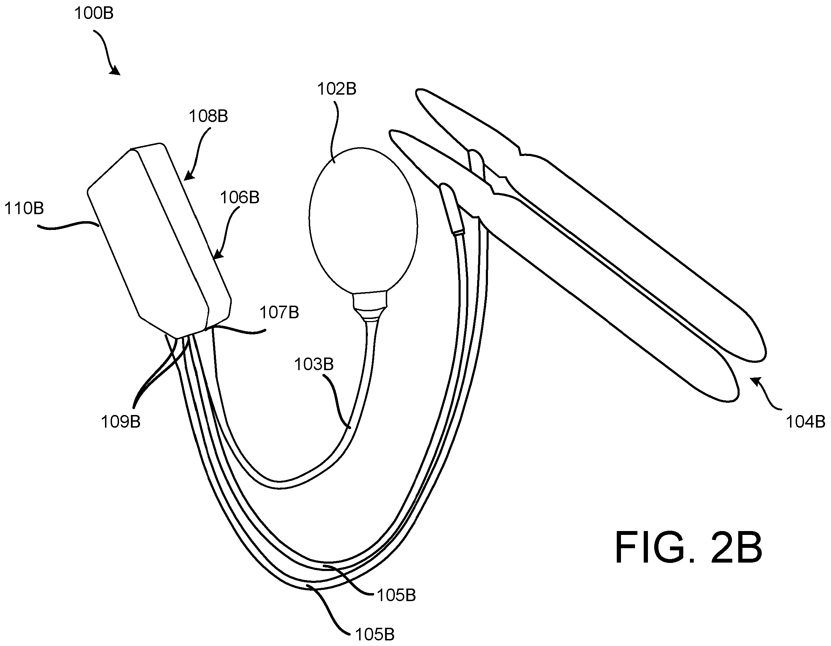

The example implantable fluid operated device 100 may be representative of a number of different types of implantable fluid operated devices. For example, the device 100 shown in FIG. 1 may be representative of an artificial urinary sphincter 100A as shown in FIG. 2A. The example artificial urinary sphincter 100A shown in FIG. 2A includes a fluid control system 106A including fluidics components such as pumps, valves and the like positioned in fluid passageways, and an electronic control system 108A configured to provide for the transfer of fluid between a reservoir 102A and an inflatable cuff 104A. Fluidics components of the fluid control system 106A, and electronic components of the electronic control system 108A may be received in a housing 110A. A first conduit 103A connects a first fluid port 107A of the fluid control system 106A/electronic control system 108A received in the housing 110A with the reservoir 102A. A second conduit 105A connects a second fluid port 109A of the fluid control system 106A/electronic control system 108A received in the housing 110A with the inflatable cuff 104A. In some examples, the device 100 shown in FIG. 1 may be representative of an inflatable penile prosthesis 100B as shown in FIG. 2B. The example penile prosthesis 100B shown in FIG. 2B includes a fluid control system 106B including fluidics components such as pumps, valves and the like positioned in fluid passageways, and an electronic control system 108B configured to provide for the transfer of fluid between a fluid reservoir 102B and inflatable cylinders 104B. Fluidics components of the fluid control system 106B, and electronic components of the electronic control system 108B may be received in a housing 110B. A first conduit 103B connects a first fluid port 107B of the fluid control system 106B/electronic control system 108B received in the housing 110B with the reservoir 102B. One or more second conduits 105B connect one or more second fluid ports 109B of the fluid control system 106A/electronic control system 108A received in the housing with the inflatable cylinders 104B. The principles to be described herein may be applied to these and other types of implantable fluid operated inflatable devices that rely on a pump assembly including various fluidics components to provide for the transfer of fluid between the different fluid filled implantable components to achieve inflation, deflation, pressurization, depressurization, deactivation and the like for effective operation. The example devices 100A, 100B shown in FIGS. 2A and 2B include electronic control systems 108A, 108B to provide for the monitoring and control of pressure and/or fluid flow through the respective devices 100A, 100B. Some of the principles to be described herein may also be applied to implantable fluid operated inflatable devices that are manually controlled.

As noted above with respect to FIG. 1, the fluid control system 106 can include a pump assembly including, for example, one or more pumps and one or more valves positioned within a fluid circuit of the pump assembly to control the transfer fluid between the fluid reservoir and the inflatable member. In some examples, the pump(s) and/or the valve(s) are electronically controlled. In some examples, the pump(s) and/or the valve(s) are manually controlled. In some examples, the pump assembly includes a fluid manifold having fluidic channels formed therein, defining the fluid circuit. In an example in which the pump assembly is electronically powered and/or controlled, the manifold may be a hermetic manifold that can contain and segment the flow of fluid from electronic components of the pump assembly, to prevent leakage and/or gas exchange. In some examples, the pump assembly includes one or more pressure sensing devices in the fluid circuit to provide for relatively precise monitoring and control of fluid flow and/or fluid pressure within the fluid circuit and/or the inflatable member. A fluid circuit configured in this manner may facilitate the proper inflation, deflation, pressurization, depressurization and deactivation of the components of the implantable fluid operated device to provide for patient safety and device efficacy.

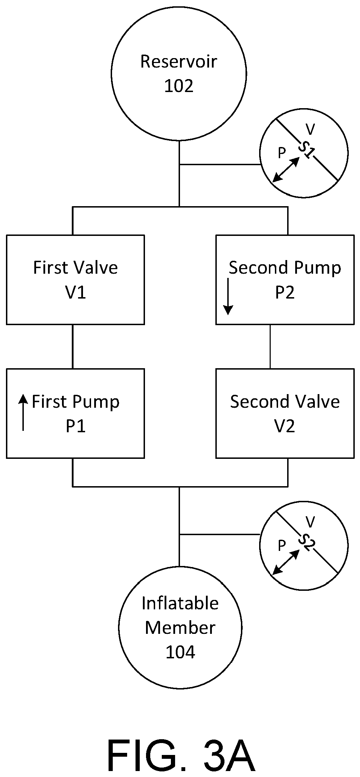

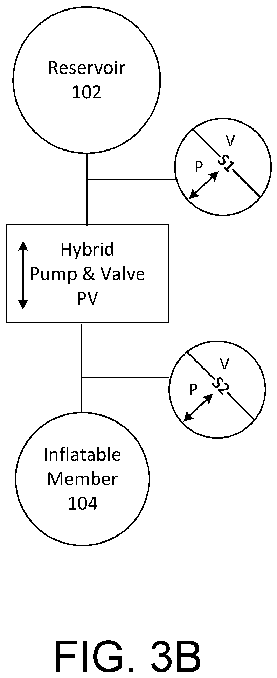

FIGS. 3A and 3B are a schematic diagrams of example fluidic architectures for an implantable fluid operated device, according to an aspect. The fluidic architecture of an implantable fluid operated device can include other orientations of fluidic channels, valve(s), pressure sensor(s) and other components than shown in FIGS. 3A and 3B. A fluidic architecture that can accommodate back pressure, pressure surges and the like enhances the performance, efficacy and efficiency of the fluid operated device 100.

The example fluidic architecture shown in FIG. 3A includes channels guiding the flow of fluid between the reservoir 102 and the inflatable member 104. In the example shown in FIG. 3A, a first valve V1 in a first fluidic channel controls the flow of fluid, generated by a first pumping device P1, from the inflatable member 104 to the reservoir 102. A second valve V2 in a second fluidic channel controls the flow of fluid, generated by a second pumping device P2, from the reservoir 102 to the inflatable member 104. In the example shown in FIG. 3A, a first pressure sensing device S1 senses a fluid pressure at the reservoir 102, and a second pressure sensing device S2 senses a fluid pressure at the inflatable member 104. The first and second pressure sensing devices S1, S2 may provide for the monitoring of fluid flow and/or fluid pressure in the fluidic channels. In the arrangement shown in FIG. 3A, one of the first pump P1 or the second pump P2 is active, while the other of the first pump P1 or the second pump P2 is in a standby mode, such that the first and second pumps do not typically operate simultaneously. For example, operation of the first pump P1 (with the second pump P2 in the standby mode) may provide for the deflation of the inflatable member 104, and operation of the second pump P2 (with the first pump P1 in the standby mode) may provide for the inflation of the inflatable member 104. The valves V1, V2 may provide for the selective sealing of the respective fluidic channel(s) so as to maintain a set state of the fluid operated device. For example, selective sealing of the respective fluidic channel(s) by the valves V1, V2 may maintain an inflated state or a deflated state of the inflatable member 104. Interaction with the valves V1, V2 (and the corresponding change in fluid flow through the fluidic architecture of the device) may change the set state of the fluid operated device. Valves V1, V2 that maintain the set state of the device until the patient requires a change in the set state of the device and initiates the required change in the set state of the device provide enhanced patient safety and improved device efficacy.

In some examples, a valve and a pump can be incorporated into a single component that provides for both the generation of fluid flow, and the control of fluid flow in the fluid operated device. In some examples, the pump can be a multi-directional pump that can pump fluid in multiple directions. The example fluidic architecture shown in FIG. 3B includes a hybrid pump and valve device PV, or a combined pump and valve device PV, including a multi-directional pumping device and one or more valves that control fluid flow through the combined pump and valve device PV. The pump and valve device PV can pump fluid in a first direction (for example, from the reservoir 102 toward the pump and valve device PV, and the pump and valve device PV toward the inflatable member 104) and in a second direction (for example, from the inflatable member 104 toward the pump and valve device PV, and the pump and valve device PV toward the reservoir 102). The incorporation of the hybrid, or combined pump and valve device PV may provide for fluid pumping and flow control in the fluid operated device using fewer components in the fluidic architecture. In some examples, this may reduce the overall size of the fluid control system including the pump assembly, any may reduce power consumption, thus increasing longevity of a power storage device, or battery, of the fluid operated device 100.

In some examples, the pump(s) P1, P2 and valve(s) V1, V2 (and/or the hybrid, or combined, pump and valve device PV) included in the fluidic architecture can each include chambers that are actuated on by a diaphragm. For example, a pump can include a first check valve at an inlet port of the pump chamber and a second check valve at an outlet port of the pump chamber, such that oscillation of the diaphragm causes forward flow (i.e. flow from the inlet port toward the outlet port). Similarly, a valve can include a valve chamber, with actuation of the diaphragm causing closure of a flow path between inlet and outlet ports of the valve chamber.

In some examples, a multi-directional pump and valve device can include a vibrating or oscillating diaphragm is positioned between a first port and a second port of a fluid chamber, with a first electronically controlled valve at the first port and a second electronically controlled valve at the second port. The first and second valves may be opened and closed in a sequence that provides for pumping in a first direction (i.e., from the first port toward the second port), for pumping in a second direction (i.e., from the second port toward the first port), or for closure of the fluid flow path. In some examples, two vibrating or oscillating diaphragms may be placed in series in a fluid flow path, for example, side to side or face to face in the fluid flow path. The first and second oscillating diaphragms act as diffusers in the flow path that sequentially restrict the flow of fluid through the flow path, in a first direction and then in a second direction. The sequentially alternating restriction of flow through the flow path in the first and second directions cause flow in either the first direction or the second direction, depending on the pattern produced by the oscillation of the first and second diaphragms and the alternating restriction of flow.

In some examples, the valves included in the fluidic architecture of a fluid operated device may be normally open valves. The use of normally open valves may provide a failsafe measure in the operation of the types of fluid operated devices as described above, particularly in a situation in which the fluid operated device is electronically controlled. For example, a failure of one of the valves within the fluid operated device, and/or an overall device failure which results in an inability to change states (for example, a sustained inflated state of the inflatable member and/or an inability to transition from an inflated state to a deflated state) may cause patient discomfort and may compromise patient safety. The use of a normally open valve and the corresponding normally open state of the valve would allow for release of pressure (for example, deflation of the inflatable member 104) and allow the fluid within the device to reach an equilibrium state, to provide for patient comfort and safety.

In some examples, one or more of the valves included in the fluidic architecture are piezoelectric valves and, in some examples, normally open piezoelectric valves. Piezoelectric materials produce electrical energy when subjected to mechanical deformation of strain. Conversely, piezoelectric materials are deformed in response to application of an electrical field. These properties allow mechanical valves to be electronically controlled through the application of voltage to the valves.

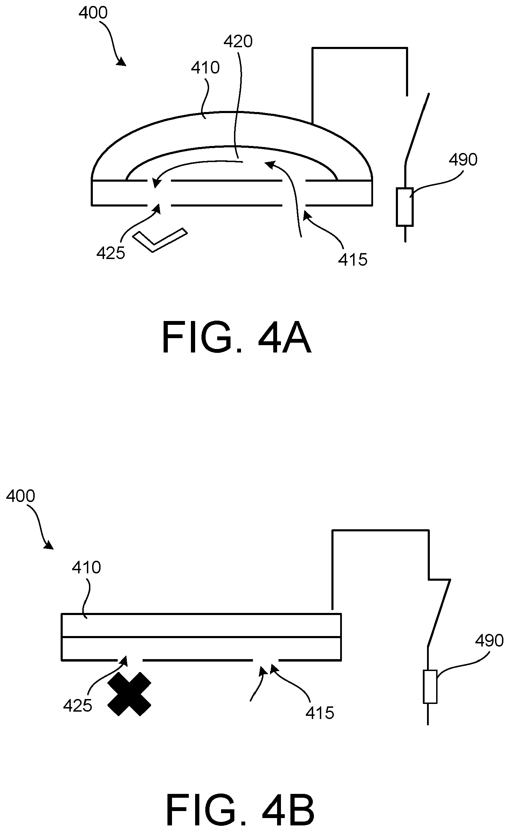

In a normally open piezoelectric valve, the valve is in the open position in the passive, or equilibrium state. The normally open valve closes in response to an application of voltage. FIG. 4A illustrates an example of normally open piezoelectric valve 400 in the equilibrium state, in which the valve 400 is open. FIG. 4B illustrates the normally open piezoelectric valve 400 in the closed state. The example normally open piezoelectric valve 400 shown in FIGS. 4A and 4B includes a piezoelectric element 410 coupled to a valve base 450. Fluid ports 415, 425 are formed in the base 450. In the example shown in FIGS. 4A and 4B, the first fluid port 415 is an inlet port 415, and the second fluid port 425 is an outlet port. A fluid chamber 420 is defined in a space between the piezoelectric element 410 and the valve base 450.

As shown in FIG. 4A, in the equilibrium state of the normally open piezoelectric valve 400 (i.e., the open state), no voltage is applied to the piezoelectric element 410 of the valve 400. In the equilibrium state in which the valve 400 is open, the piezoelectric element 410 of the valve 400 is deformed or deflected, allowing fluid to flow into the chamber 420 through the first port 415, and out of the chamber 420 through the second port 425. Application of a voltage to the piezoelectric element 410 of the valve 400 causes the valve 400 to close, as shown in FIG. 4B. In some examples, an electrical bias may remain in the piezoelectric element 410 after removal of the voltage. The electrical bias accumulated in the piezoelectric element 410 of the valve may dissipate over time, maintaining the closed state of the valve 400 through at least a portion of the dissipation period. In some examples, a resistor 490 may be positioned in the circuit, in parallel to the piezoelectric element 410. The resistor 490 may provide for the controlled dissipation of voltage accumulated in the piezoelectric element 410, so that the normally open valve 400 is returned to the equilibrium/open state shown in FIG. 4A in a time-controlled manner.

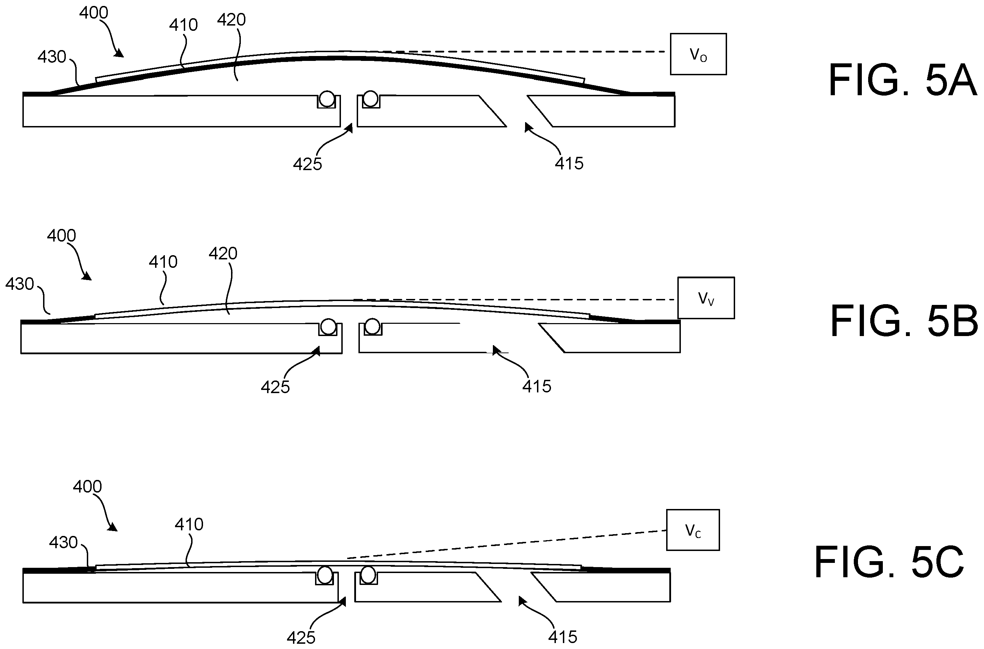

In some examples, a voltage level applied to the valve 400 may be varied, to, for example, vary an opening amount of the valve 400, vary a flow rate through the valve 400 and the like. In some examples, variable voltage control may be applied to account for changes in atmospheric pressure (for example, changes in altitude or depth) which affect pressure of fluid flowing in the fluid operated device, and thus can affect the proper operation of the fluid operated device. FIG. 5A illustrates a state of the valve 400 in which a fully open voltage Vo is applied to the piezoelectric element 410 of the valve 400 such that the valve 400 is in a fully open state. FIG. 5B illustrates a state of the valve 400 in which a partially open voltage Vv, or variable voltage Vv is applied to the valve 400 such that the valve 400 is in a partially open state. FIG. 5C illustrates a state of the valve 400 in which a fully closed voltage Vc is applied to the valve 400 such that the valve 400 is in a fully closed state. In a case in which the valve 400 is the normally open piezoelectric valve 400 described above, the normal, equilibrium state is the open state shown in FIG. 5A, and thus the fully open voltage Vo would be essentially zero.

In some examples, as the fluid operated device experiences changes in atmospheric and/or working pressure (due to, for example, changes in altitude and/or depth), a corresponding change in fluid pressure and/or fluid flow rate through the valve 400 may be experienced. Without adjustment, these changes in fluid pressure and/or fluid flow rate may impact (adversely impact) the proper operation of the fluid operated device.

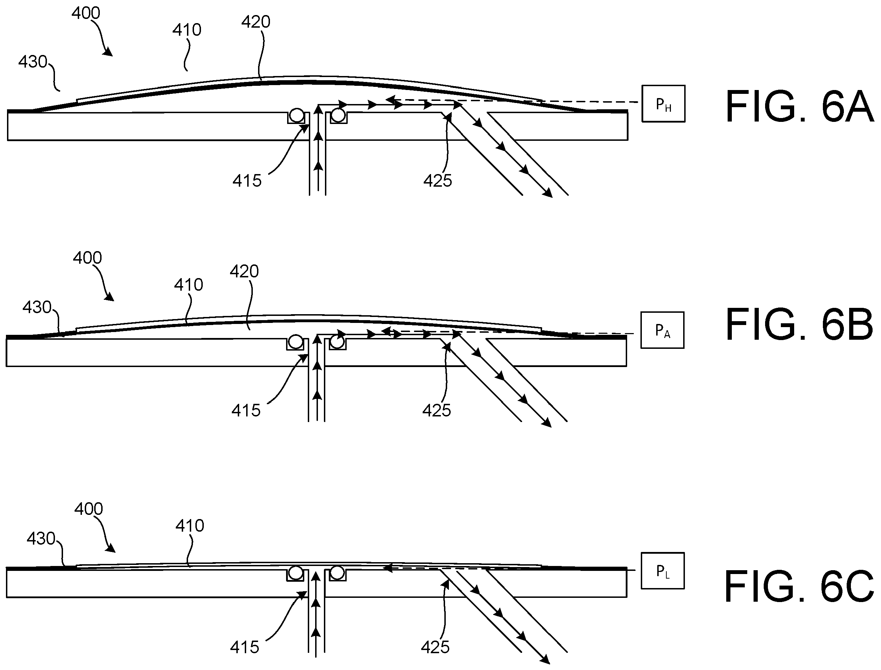

FIG. 6A illustrates the piezoelectric valve 400 in a state in which a pressure P_{H} in the chamber 420 and fluid passageways is relatively high compared to the pressure outside of the device (i.e., atmospheric and/or working pressure), thus drawing the piezoelectric element 410 and diaphragm 430 away from the chamber 420 and increasing a flow rate through the valve 400. FIG. 6B illustrates the piezoelectric valve 400 in a state in which the pressure P_{A} in the chamber 420 and fluid passageways is essentially the same as outside of the device. FIG. 6C illustrates the piezoelectric valve 400 in a state in which the pressure P_{L} in the chamber 420 and fluid passageways is measurably less than outside of the device, thus pulling the piezoelectric element 410 and diaphragm 430 toward the chamber 420 close off the fluid passageways and restrict fluid flow through the valve 400.

As noted above, the fluid operated device may experience varying levels of atmospheric and/or working pressure, which changes with, for example, altitude or depth. For example, atmospheric and/or working pressure decreases as altitude increases, and thus a patient having an implanted fluid operated device may experience decreased atmospheric and/or working pressure when flying. The decreased atmospheric and/or working pressure may affect operation of the piezoelectric valve 400 as shown in FIG. 6A. That is, the decreased atmospheric and/or working pressure may cause the baseline position of the diaphragm 430 to change as shown, causing an increased fluid flow rate through the valve 400. A patient having an implanted fluid operated device may experience increased atmospheric and/or working pressure when, for example, submerged or swimming in water. The increased atmospheric and/or working pressure may affect operation of the piezoelectric valve 400 as shown in FIG. 6C. That is, the increased atmospheric and/or working pressure may cause the baseline position of the diaphragm 430 to change as shown, causing a decrease in fluid flow rate through the valve 400, or restricting flow through the valve 400. Without correction/recalibration for the movement of the diaphragm 430 in response to the variation in atmospheric and/or working pressure, either of these situations may result in improper operation of the fluid operated device, to the point where patient comfort and safety could be impacted.

An electronically controlled fluid operated device has the ability to obtain atmospheric and/or working pressure substantially real time. The electronically controlled fluid operated device may use detected atmospheric and/or working pressure levels, and detected changes in atmospheric and/or working pressure levels, to adjust pressure levels in the fluid operated device, and in particular to adjust operation of flow control valves in the fluid operated device (for example, open/close level of the valves), to ensure proper fluid pressure levels and fluid flow rates for safe operation of the fluid operated device.

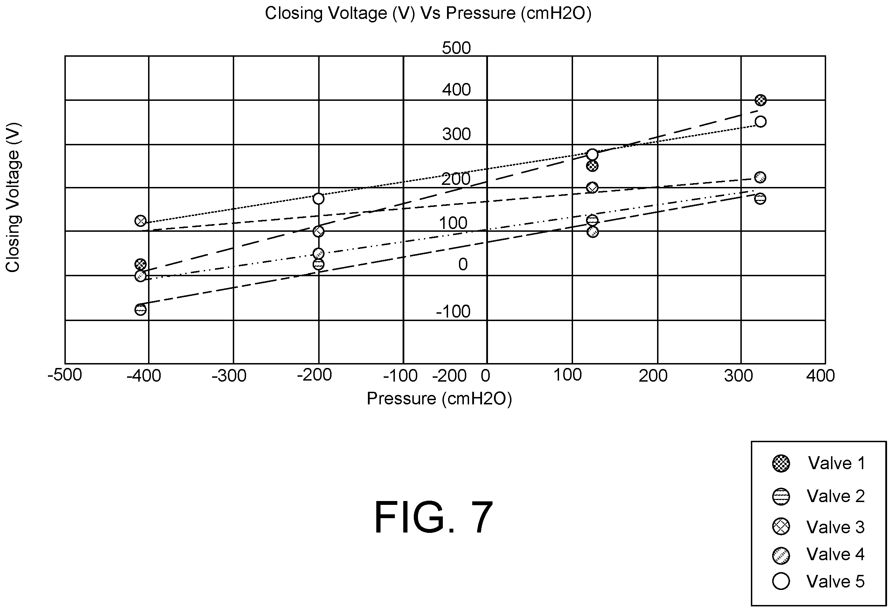

In some examples, a variable closing voltage may be applied to one or more of the valves 400 in the fluid operated device to provide a corrected level of closure of the valve 400 corresponding to the varying levels of atmospheric and/or working pressure the valve 400 may experience, based on for example, altitude or depth. In some examples, a calibration curve may be referenced to determine an appropriate closing voltage for a given atmospheric and/or working pressure sensed by the electronically controlled fluid operated device. An example calibration curve is shown in FIG. 7. The example calibration curve shown in FIG. 7 illustrates the voltage required to close a fluid passageway at different atmospheric and/or working pressures for a particular valve.

In some examples, a calibration curve for each valve 400 of the fluid operated device can be stored, for example, in the form of a look up table, in a memory of the electronic control system 108. During operation, as atmospheric and/or working pressure (and changes in atmospheric and/or working pressure) is sensed, the electronic control system 108 can access the appropriate calibration curve/look up table for each of the valves 400, and adjust a voltage level applied to each of the valves 400 accordingly, to maintain a current state of the fluid operated device.

The ability to determine variable closing voltages for each of the flow control valves within the fluid operated device to account for varying atmospheric conditions (for example, as pressure varies with altitude and/or depth) and to adjust applied voltages accordingly to maintain a current state of the fluid operated device may provide for the proper operation of the fluid operated device even when subjected to varying atmospheric conditions. This may improve patient comfort and safety considerations. The risk of over-driving the piezoelectric element, and adversely impacting overall device performance, may be greatly reduced, particularly in a high altitude (low atmospheric and/or working pressure) situation, in which the pressure in the fluid passageways is relatively low. The ability to apply an adjusted closing voltage (for example, a lower closing voltage) may improve reliability of the fluid operated device by reducing or substantially eliminating leakage issues in response to increased pressure levels in the fluid passageways at depths and reduced pressure levels in the fluid passageways at altitudes. Improved, and even optimum, sealing may be achieved by applying an adjusted voltage to the valve 400 at a particular atmospheric condition, thus providing for the proper fluid flow volume and rate through the valve 400 and avoiding damage to the valve 400. The proper sealing of the fluid passageways at varying atmospheric conditions afforded by the use of the calibration curves to determine proper closing voltages for the valves 400 may guard against over-pressuring of the inflatable member, reduce risk of piezo damage thus further enhancing patient safety and comfort.

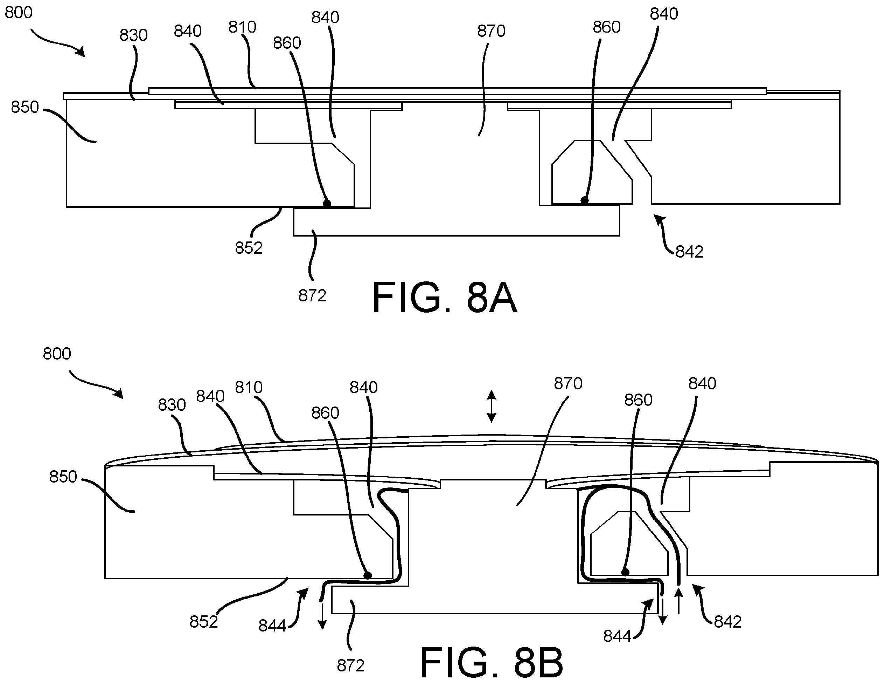

In some examples, the fluidic architecture of a fluid operated device may include one or more normally closed valves. A normally closed valve may provide for maximum sealing without the need for activation (such as, for example the application of voltage), which may be advantageous in some positions within the fluid operated device, and in some situations. FIG. 8A illustrates an example normally closed valve 800 in closed state. FIG. 8B illustrates the example normally closed valve 800 in an open state.

The example normally closed valve 800 shown in FIGS. 8A and 8B includes a plunger 870 movably positioned with respect to a valve base 850 so as to selectively block a fluid passageway 880 defined in the valve base 850. In the closed state shown in FIG. 8A, a flange 872 of the plunger 870 is positioned against a sealing surface 852 of the valve base 850, with an O-ring 860 positioned in a sealing notch formed in the valve base 850. In some examples, the O-ring may be positioned in a sealing notch formed in the flange 872 of the plunger 870. A piezoelectric element 810 is mounted on an external foil 830 coupled to the valve base 850. An internal foil 840 is fixed to the plunger 870 and to the base 850.

In the closed state of the valve 800 shown in FIG. 8A, the piezoelectric element 810 has not been actuated (i.e., a voltage has not been applied), and the flange 872 of the plunger 870 is positioned against the sealing surface 852 of the valve base 850, thus forming a seal that blocks the fluid passageway 880. To change the state of the normally closed valve 800 from the closed state to the open state shown in FIG. 8B, a voltage is applied to the piezoelectric element 810, causing a deflection of the piezoelectric element 810, the external foil 830, and the internal foil 840. In particular, the application of voltage to the piezoelectric element 810 has caused an upward (in the example orientation shown in FIG. 8B) of the piezoelectric element 810 mounted on the external foil 830, and a downward deflection of the internal foil 840 attached to the valve base 850 and the plunger 870.This downward deflection of the internal foil 840 drives the plunger 870 downward together with the internal foil 840. Deflection of the piezoelectric element 810 and the external and internal foils 830, 840 and movement of the plunger 870 in this manner allows fluid to flow through the valve 800 as shown in FIG. 8B. That is, in the open state shown in FIG. 8B, the plunger 870 has moved downward (in the example orientation shown in FIGS. 8A and 8B), away from the valve base 850, such that a space is formed between the flange 872 of the plunger 870 and the sealing surface 852 of the of the valve base 850. This movement releases the seal between the plunger 870 and the valve base 850, allowing fluid to flow into the fluid passageway 880 through at least one inlet 842, and out of the fluid passageway 880 through one or more outlets 844. In some examples, cyclic application and release of voltage applied to the piezoelectric element 810 may generate reciprocal movement of the plunger 870 as the plunger 870 alternates between the open position shown in FIG. 8A and the closed position shown in FIG. 8B.

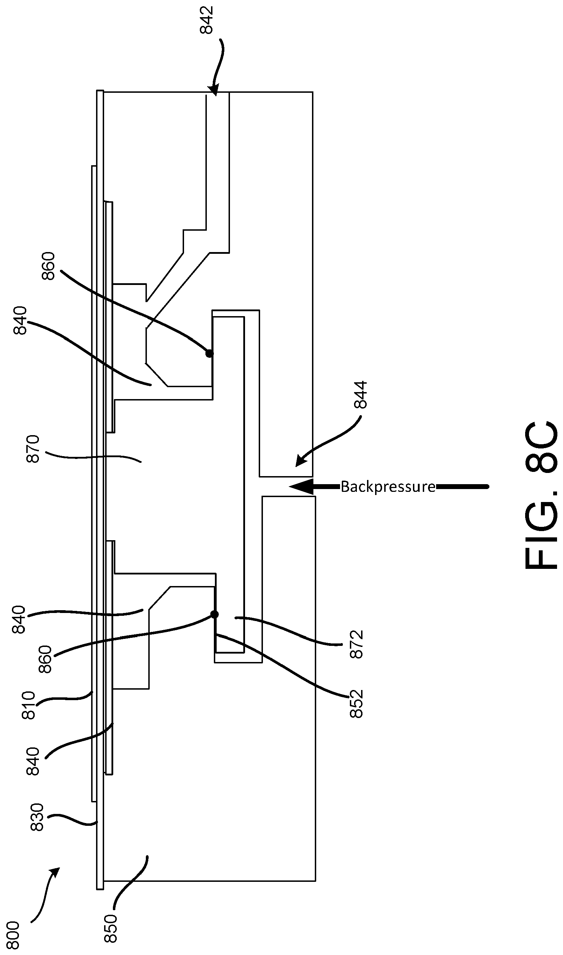

In some situations, the normally closed valve 800 may experience a sudden surge in pressure of the fluid due to, for example, a fall, physical exertion and the like. The sudden surge or spike in pressure may cause the plunger 870 to move, resulting in leakage through the valve 800 as the valve 800 is forced from the closed position to the open position. Application of a back pressure at the outlet 844 of the normally closed valve 800 as shown in FIG. 8C will urge the plunger 870 into engagement against the valve base 850 thus increasing sealing pressure in the valve 800. The increased sealing pressure will reduce the risk of leaking in the event of a sudden surge or spike of fluid pressure in the valve 800, and will guard against an unwanted change of state of the valve 800.

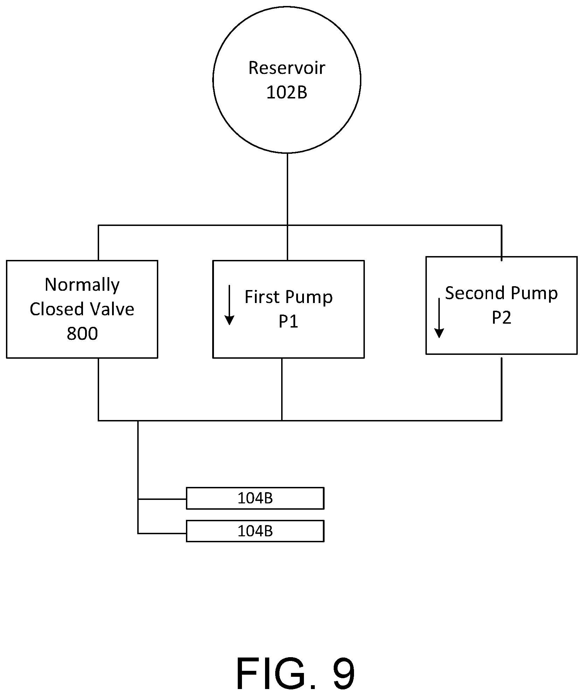

The schematic diagram shown in FIG. 9 illustrates an example fluid architecture for a fluid operated device in the form of the inflatable penile prosthesis 100B described above. In this example arrangement, the inflatable cylinders 104B are in-line with the normally closed valve 800. Thus, when a pressure surge or spike is experienced in the cylinders 104B, the pressure spike could be harnessed to apply the back pressure to the normally closed valve 800 as described above. This may provide for increased sealing pressure in the valve 800 during the pressure spike, thus avoiding leakage of fluid through the valve 800 and maintaining the desired state of the valve 800 and the cylinders 104B.

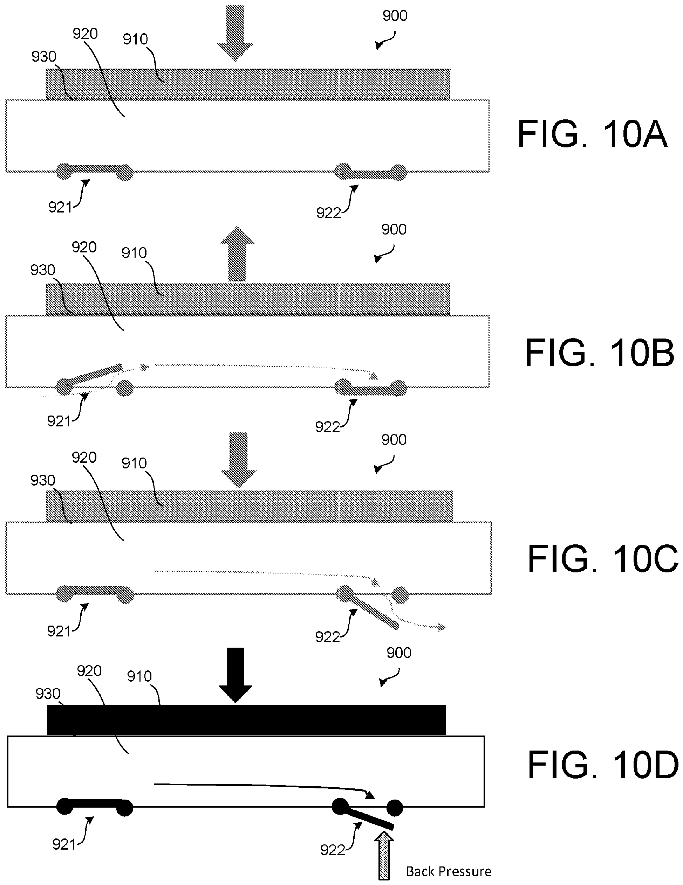

FIGS. 10A-10D illustrate a hybrid, or combined pump and valve device 900 as described above, in which back pressure is applied in response to a detected surge or spike in pressure. The pump and valve device 900 includes a piezoelectric element 910 positioned on a diaphragm 930 along a side of a fluid chamber 920 of the pump and valve device 900. A first check valve 921 is positioned at a first end of the chamber 920, for example, an inlet end of the chamber 920, corresponding to a first end portion of the piezoelectric element 910. The first check valve 921 regulates fluid flow in a first direction, for example, into the chamber 920. A second check valve 922 is positioned at a second end of the chamber 920, for example, an outlet end of the chamber 920, corresponding to a second end portion of the piezoelectric element 910. The second check valve 922 regulates flow in a second direction, for example out of the chamber 920.

A supply stroke, or up stroke, of the pump and valve device 900 (in which the piezoelectric element 910 moves from the concave position shown in FIG. 10A to the convex position shown in FIG. 10B) and the corresponding pressure differential draws fluid into the chamber 920 through the first check valve 921, while the second check valve 922 remains closed. A pressure stroke, or down stroke, of the pump and valve device, including contraction of the piezoelectric element 910, from the convex position shown in FIG. 10B to the concave position shown in FIG. 10C, closes the first check valve 921 and allows fluid to flow out of the chamber 920 through the second check valve 922. The pumping cycle can be repeated to continue to pump fluid into and out of, or through the chamber 920. In this arrangement, application of a backpressure, as shown in FIG. 10D, may provide for forced sealing of the first and second check valves 921, 922 in the event of a spike or surge in fluid pressure that would otherwise result in an unwanted change of state of the fluid operated device.

The invention is defined by the claims.

## Claims

1. An implantable fluid operated inflatable device (100), comprising:
a fluid reservoir (102);
an inflatable member (104);
a fluid control system (106) configured to transfer fluid between the fluid reservoir (102) and the inflatable member (104), including:
a housing (110A, 110B);
at least one valve (V1, V2) and at least one pump (P1, P2) positioned in a fluid passageway within in the housing (110A, 110B);
a first fluid port (107A, 107B) in fluidic communication with the fluid reservoir (102); and
a second fluid port (109A, 109B) in fluidic communication with the inflatable member (104);
at least one pressure sensing device (S1, S2) configured to sense a fluid pressure in the implantable fluid operated inflatable device (100); and
an electronic control system (108) configured to receive the pressure sensed by the at least one pressure sensing device (S1, S2), and to control the at least one valve (V1, V2) and at least one pump (P1, P2) in response to the received pressure,
**characterized in that** the at least one pump is a piezoelectric pump and valve device, including:
a valve base (450);
at least one inlet port (415) formed in the valve base (450);
at least one outlet port (425) formed in the valve base (450);
a diaphragm (430) coupled to the valve base (450); and
a piezoelectric element (410) mounted on the diaphragm (430),
and **in that** a voltage applied to the piezoelectric element (410) is a variable voltage to maintain a set state of the fluid operated inflatable device (100) based on a pressure detected in the fluid passageway of the valve relative to a detected pressure external to the valve.

2. The implantable fluid operated inflatable device (100) of claim 1, wherein the at least one pump (P1, P2) includes a combined pump and valve device positioned inline between the reservoir (102) and the inflatable member (104), including:
a chamber (920);
a diaphragm (930) positioned along an edge portion of the chamber (920);
a piezoelectric element (910) mounted on the diaphragm (930);
a first valve (921) positioned at a first end portion of the chamber (920) corresponding to a first end portion of the piezoelectric element (910); and
a second valve (922) positioned at a second end portion of the chamber (920) corresponding to a second end portion of the piezoelectric element .

3. The implantable fluid operated inflatable device (100) of claim 2, wherein
in a first mode in which fluid is moved through the combined pump and valve device in a first direction to transfer fluid from the reservoir (102) to the inflatable member (104) to inflate the inflatable member (104), a first pumping cycle of the combined pump and valve device includes:
a first supply stroke in which fluid is drawn into the chamber (920) through the first valve (921) while the second valve (922) is closed; and
a first pressure stroke in which fluid is expelled out of the chamber (920) through the second valve (922) while the first valve (921) is closed; and
in a second mode in which fluid is moved through the combined pump and valve device in a second direction to transfer fluid from the inflatable member (104) to the reservoir (102) to deflate the inflatable member (104), a second pumping cycle of the combined pump and valve device includes:
a second supply stroke in which fluid is drawn into the chamber (920) through the second valve (922) while the first valve (921) is closed; and
a second pressure stroke in which fluid is expelled out of the chamber through the first valve (921) while the second valve (922) is closed.

4. The implantable fluid operated inflatable device (100) of claim 3, wherein
the first supply stroke and the first pressure stroke are alternately and repeatedly implemented until an inflation pressure of the inflatable member (104) is achieved based on a pressure sensed by the at least one sensing device (S1, S2); and
the second supply stroke and the second pressure stroke are alternately and repeatedly implemented until a deflation pressure is achieved based on a pressure sensed by the at least one sensing device (S1, S2).

5. The implantable fluid operated inflatable device (100) of any of claims 1 to 4, wherein the variable voltage applied to the piezoelectric element (410) to maintain the set state of the fluid operated inflatable device (100) is based on the pressure detected in the fluid passageway of the valve relative to an atmospheric pressure sensed by the electronic control system (108).

6. The implantable fluid operated inflatable device (100) of any of claims 1 to 5, wherein the variable voltage applied to the piezoelectric element (410) adjusts a position of the piezoelectric element (410) and the diaphragm (430) so as to adjust at least one of a fluid pressure or a fluid flow rate to adjust for atmospheric conditions and correspond to the set state of the fluid-controlled inflatable device.

7. The implantable fluid operated inflatable device (100) of any of claims 1 to 6, wherein the voltage applied to the piezoelectric element (410) is selected from a calibration curve associated with the piezoelectric valve that is accessible in a memory of the electronic control system (108).

8. The implantable fluid operated inflatable device (100) of any of claims 1 to 7, wherein the pump and valve device includes a normally open piezoelectric valve (400) that is configured to transition from a normally open state to a closed state in response to an application of voltage to the piezoelectric element (410), and to return to the normally open state in response to release of the voltage.

9. The implantable fluid operated inflatable device (100) of claim 8, wherein the normally open piezoelectric valve (400) is configured to remain in the closed state for a period of time after release of the voltage, and to transition to the normally open state in response to dissipation of electrical bias accumulated in the piezoelectric element (410).

10. The implantable fluid operated inflatable device (100) of claim 8 or 9, the normally open piezoelectric valve (400) further comprising a resistor (490) electrically connected to the piezoelectric element (410), wherein the resistor (490) is configured to control a dissipation of electrical bias accumulated in the piezoelectric element (410) such that the normally open piezoelectric valve (400) transitions from the closed state to the normally open state in a set period of time after release of the voltage.

11. The implantable fluid operated inflatable device (100) of any of claims 1 to 10, wherein the piezoelectric valve is a normally closed piezoelectric valve (800) that is configured to transition from a normally closed state to an open state in response to an application of voltage to the piezoelectric element (810), and to return to the normally closed state in response to release of the voltage, the normally closed piezoelectric valve (800) including:
a plunger (870) movably positioned within the fluid passageway of the normally closed piezoelectric valve (800), wherein the plunger (870) is sealed against the valve base in the normally closed state so as to restrict flow through the fluid passageway, and is spaced apart from the valve base in the open state so as to open the fluid passageway.

12. The implantable fluid operated inflatable device (100) of claim 11, wherein, in the normally closed state of the normally closed piezoelectric valve (800), a backpressure applied to the plunger (870) through the at least one outlet maintains the sealed position of the plunger (870) against the valve base in response to a surge in fluid pressure at the at least one inlet (842).

13. The implantable fluid operated inflatable device (100) of any of claims 1 to 12, wherein the electronic control system (108) includes a printed circuit board including a memory configured to store at least one control algorithm, a communication module configured to communicate with one or more external devices, and a processor configured to:
receive pressure level measurements from the at least one sensing device;
apply the at least one control algorithm based on the received pressure level measurements; and
control operation of the at least one valve and the at least one pump in accordance with the applied at least one control algorithm.

14. The implantable fluid operated device (100) of any of claims 1 to 13, wherein the implantable fluid operated inflatable device (100) is an artificial urinary sphincter (100A) or an inflatable penile prosthesis (100B).

## Patentansprüche

1. Implantierbare, fluidbetriebene, fluidexpandierbare Vorrichtung (100), die umfasst:
ein Fluidreservoir (102);
ein fluidexpandierbares Element (104);
ein Fluidsteuerungssystem (106), das eingerichtet ist, Fluid zwischen dem Fluidreservoir (102) und dem fluidexpandierbaren Element(104) zu transferieren, enthaltend:
ein Gehäuse (110A, 110B)
mindestens ein Ventil (V1, V2) und mindestens eine Pumpe (P1, P2), die in einem Fluiddurchgang innerhalb des Gehäuses (110A, 110B) angeordnet sind;
eine erste Fluidöffnung (107A, 107B) in Fluidverbindung mit dem Fluidreservoir (102); und
eine zweite Fluidöffnung (109A, 109B), die in Fluidverbindung mit dem fluidexpandierbaren Element (104) steht;
mindestens eine Druckerfassungsvorrichtung (S1, S2), die eingerichtet ist, einen Fluiddruck in der implantierbaren fluidbetriebenen fluidexpandierbaren Vorrichtung (100) zu messen; und
ein elektronisches Steuersystem (108), das eingerichtet ist, den von der mindestens einen Druckerfassungsvorrichtung (S1, S2) gemessenen Druck zu empfangen und das mindestens eine Ventil (V1, V2) und die mindestens eine Pumpe (P1, P2) in Reaktion auf den empfangenen Druck zu steuern,
**dadurch gekennzeichnet, dass** die mindestens eine Pumpe eine piezoelektrische Pump- und Ventilvorrichtung ist, die enthält:
eine Ventilbasis (450);
mindestens eine Einlassöffnung (415), die in der Ventilbasis (450) ausgebildet ist;
mindestens eine Auslassöffnung (425), die in der Ventilbasis (450) ausgebildet ist;
eine Membran (430), die mit der Ventilbasis (450) gekoppelt ist; und
ein piezoelektrisches Element (410), das auf der Membran (430) angebracht ist,
und dass eine an das piezoelektrische Element (410) angelegte Spannung eine variable Spannung ist, um einen eingestellten Zustand der fluidbetriebenen fluidexpandierbaren Vorrichtung (100) auf der Grundlage eines im Fluiddurchgang des Ventils erfassten Drucks relativ zu einem erfassten Druck außerhalb des Ventils aufrechtzuerhalten.

2. Implantierbare, fluidbetriebene, fluidexpandierbare Vorrichtung (100) nach Anspruch 1, wobei die mindestens eine Pumpe (P1, P2) eine kombinierte Pumpen- und Ventilvorrichtung umfasst, die in Reihe zwischen dem Reservoir (102) und dem fluidexpandierbaren Element (104) angeordnet ist, enthaltend:
eine Kammer (920);
eine Membran (930), die entlang eines Randbereichs der Kammer (920) angeordnet ist;
ein piezoelektrisches Element (910), das auf der Membran (930) angebracht ist;
ein erstes Ventil (921), das an einem ersten Endteil der Kammer (920) angeordnet ist, das einem ersten Endteil des piezoelektrischen Elements (910) entspricht; und
ein zweites Ventil (922), das an einem zweiten Endteil der Kammer (920) angeordnet ist, das einem zweiten Endteil des piezoelektrischen Elements entspricht.

3. Implantierbare, fluidbetriebene, fluidexpandierbare Vorrichtung (100) nach Anspruch 2, wobei
in einem ersten Modus, in dem Fluid durch die kombinierte Pumpen- und Ventilvorrichtung in einer ersten Richtung bewegt wird, um Fluid von dem Reservoir (102) zu dem fluidexpandierbaren Element (104) zu übertragen, um das fluidexpandierbare Element (104) zu expandieren, ein erster Pumpzyklus der kombinierten Pumpen- und Ventilvorrichtung umfasst:
einen ersten Zufuhrhub, bei dem Flüssigkeit durch das erste Ventil (921) in die Kammer (920) gesaugt wird, während das zweite Ventil (922) geschlossen ist; und
einen ersten Druckhub, bei dem Fluid aus der Kammer (920) durch das zweite Ventil (922) ausgestoßen wird, während das erste Ventil (921) geschlossen ist; und
in einem zweiten Modus, in dem Fluid durch die kombinierte Pumpen- und Ventilvorrichtung in einer zweiten Richtung bewegt wird, um Fluid von dem fluidexpandierbaren Element (104) zu dem Reservoir (102) zu transferieren, um das fluidexpandierbare Element (104) zu entleeren, ein zweiter Pumpzyklus der kombinierten Pumpen- und Ventilvorrichtung umfasst:
einen zweiten Zufuhrhub, bei dem Flüssigkeit durch das zweite Ventil (922) in die Kammer (920) gesaugt wird, während das erste Ventil (921) geschlossen ist; und
einen zweiten Druckhub, bei dem Flüssigkeit aus der Kammer durch das erste Ventil (921) ausgestoßen wird, während das zweite Ventil (922) geschlossen ist.

4. Implantierbare, fluidbetriebene, fluidexpandierbare Vorrichtung (100) nach Anspruch 3, wobei
der erste Zufuhrhub und der erste Druckhub abwechselnd und wiederholt ausgeführt werden, bis ein Expansionsdruck des fluidexpandierbaren Elements (104) auf der Grundlage eines von der mindestens einen Erfassungsvorrichtung (S1, S2) erfassten Drucks erreicht ist; und
der zweite Zufuhrhub und der zweite Druckhub abwechselnd und wiederholt ausgeführt werden, bis ein Entleerungsdruck auf der Grundlage eines von der mindestens einen Erfassungsvorrichtung (S1, S2) erfassten Drucks erreicht ist.

5. Implantierbare, fluidbetriebene, fluidexpandierbare Vorrichtung (100) nach einem der Ansprüche 1 bis 4, wobei die variable Spannung, die an das piezoelektrische Element (410) angelegt wird, um den eingestellten Zustand der fluidbetriebenen, fluidexpandierbaren Vorrichtung (100) beizubehalten, auf dem Druck basiert, der in dem Fluiddurchgang des Ventils relativ zu einem atmosphärischen Druck erfasst wird, der von dem elektronischen Steuersystem (108) erfasst wird.

6. Implantierbare, fluidbetriebene, fluidexpandierbare Vorrichtung (100) nach einem der Ansprüche 1 bis 5, wobei die an das piezoelektrische Element (410) angelegte variable Spannung eine Position des piezoelektrischen Elements (410) und der Membran (430) einstellt, um zumindest einen Fluiddruck oder eine Fluiddurchflussrate einzustellen, um sich an atmosphärische Bedingungen anzupassen und dem eingestellten Zustand der fluidgesteuerten, fluidexpandierbaren Vorrichtung zu entsprechen.

7. Implantierbare fluidbetriebene fluidexpandierbare Vorrichtung (100) nach einem der Ansprüche 1 bis 6, wobei die an das piezoelektrische Element (410) angelegte Spannung aus einer dem piezoelektrischen Ventil zugeordneten Kalibrierungskurve ausgewählt wird, die in einem Speicher des elektronischen Steuersystems (108) zugänglich ist.

8. Implantierbare, fluidbetriebene, fluidexpandierbare Vorrichtung (100) nach einem der Ansprüche 1 bis 7, wobei die Pump- und Ventilvorrichtung ein normalerweise offenes piezoelektrisches Ventil (400) umfasst, das eingerichtet ist, als Reaktion auf das Anlegen einer Spannung an das piezoelektrische Element (410) von einem normalerweise offenen Zustand in einen geschlossenen Zustand zu übergehen und als Reaktion auf das Aufheben der Spannung in den normalerweise offenen Zustand zurückzukehren.

9. Implantierbare, fluidbetriebene, fluidexpandierbare Vorrichtung (100) nach Anspruch 8, wobei das normalerweise offene piezoelektrische Ventil (400) eingerichtet ist, nach dem Aufheben der Spannung für eine Zeitspanne in dem geschlossenen Zustand zu verbleiben und als Reaktion auf die Dissipation der in dem piezoelektrischen Element (410) angesammelten elektrischen Vorspannung in den normalerweise offenen Zustand überzugehen.

10. Implantierbare, fluidbetriebene, fluidexpandierbare Vorrichtung (100) nach Anspruch 8 oder 9, wobei das normalerweise offene piezoelektrische Ventil (400) ferner einen Widerstand (490) umfasst, der elektrisch mit dem piezoelektrischen Element (410) verbunden ist, wobei der Widerstand (490) eingerichtet ist, eine Dissipation der in dem piezoelektrischen Element (410) akkumulierten elektrischen Vorspannung derart zu steuern, dass das normalerweise offene piezoelektrische Ventil (400) in einer festgelegten Zeitspanne nach dem Aufheben der Spannung von dem geschlossenen Zustand in den normalerweise offenen Zustand übergeht.

11. Implantierbare, fluidbetriebene, fluidexpandierbare Vorrichtung (100) nach einem der Ansprüche 1 bis 10, wobei das piezoelektrische Ventil ein normalerweise geschlossenes piezoelektrisches Ventil (800) ist, das eingerichtet ist, als Reaktion auf das Anlegen einer Spannung an das piezoelektrische Element (810) von einem normalerweise geschlossenen Zustand in einen offenen Zustand überzugehen und als Reaktion auf das Aufheben der Spannung in den normalerweise geschlossenen Zustand zurückzukehren, wobei das normalerweise geschlossene piezoelektrische Ventil (800) umfasst:
einen Kolben (870), der beweglich innerhalb des Fluiddurchgangs des normalerweise geschlossenen piezoelektrischen Ventils (800) positioniert ist, wobei der Kolben (870) im normalerweise geschlossenen Zustand gegen die Ventilbasis abgedichtet ist, um den Fluss durch den Fluiddurchgang zu beschränken, und im offenen Zustand von der Ventilbasis beabstandet ist, um den Fluiddurchgang zu öffnen.

12. Implantierbare, fluidbetriebene, fluidexpandierbare Vorrichtung (100) nach Anspruch 11, wobei im normalerweise geschlossenen Zustand des normalerweise geschlossenen piezoelektrischen Ventils (800) ein Gegendruck, der auf den Kolben (870) durch den mindestens einen Auslass ausgeübt wird, die abgedichtete Position des Kolbens (870) gegen die Ventilbasis als Reaktion auf einen Anstieg des Fluiddrucks an dem mindestens einen Einlass (842) aufrechterhält.

13. Implantierbare, fluidbetriebene, fluidexpandierbare Vorrichtung (100) nach einem der Ansprüche 1 bis 12, wobei das elektronische Steuersystem (108) eine gedruckte Schaltung mit einem Speicher, der zum Speichern mindestens eines Steueralgorithmus eingerichtet ist, ein Kommunikationsmodul, das zur Kommunikation mit einer oder mehreren externen Vorrichtungen eingerichtet ist, und einen Prozessor enthält, der dazu eingerichtet ist:
Druckpegelmessungen von der mindestens einen Erfassungsvorrichtung zu empfangen;
den mindestens einen Steuerungsalgorithmus auf der Grundlage der empfangenen Druckpegelmessungen anzuwenden; und
den Betriebs des mindestens einen Ventils und der mindestens einen Pumpe in Übereinstimmung mit dem angewandten mindestens einen Steuerungsalgorithmus zu steuern.

14. Implantierbare, fluidbetriebene Vorrichtung (100) nach einem der Ansprüche 1 bis 13, wobei die implantierbare, fluidbetriebene, fluidexpandierbare Vorrichtung (100) ein künstlicher Harnröhrenschließmuskel (100A) oder eine fluidexpandierbare Penisprothese (100B) ist.

## Revendications

1. Dispositif gonflable actionné par fluide implantable (100), comprenant :
un réservoir de fluide (102) ;
un élément gonflable (104) ;
un système de commande de fluide (106) configuré pour transférer du fluide entre le réservoir de fluide (102) et l'élément gonflable (104), incluant :
un boîtier (110A, 110B) ;
au moins une soupape (V1, V2) et au moins une pompe (P1, P2) qui sont positionnées dans un passage de fluide à l'intérieur du boîtier (110A, 110B) ;
un premier orifice de fluide (107A, 107B) en communication fluidique avec le réservoir de fluide (102) ; et
un second orifice de fluide (109A, 109B) en communication fluidique avec l'élément gonflable (104) ;
au moins un dispositif de détection de pression (S1, S2) configuré pour détecter une pression de fluide dans le dispositif gonflable actionné par fluide implantable (100) ; et
un système de commande électronique (108) configuré pour recevoir la pression qui est détectée par l'au moins un dispositif de détection de pression (S1, S2) et pour commander l'au moins une soupape (V1, V2) et au moins une pompe (P1, P2) en réponse à la pression reçue,
**caractérisé en ce que** l'au moins une pompe est un dispositif à pompe(s) et soupape(s) piézoélectriques, incluant :
une base de soupape (450) ;
au moins un orifice d'entrée (415) qui est formé dans la base de soupape (450) ;
au moins un orifice de sortie (425) qui est formé dans la base de soupape (450) ;
un diaphragme (430) qui est couplé à la base de soupape (450) ; et
un élément piézoélectrique (410) qui est monté sur le diaphragme (430),
et **en ce que** une tension qui est appliquée sur l'élément piézoélectrique (410) est une tension variable afin de maintenir un état défini du dispositif gonflable actionné par fluide (100) sur la base d'une pression qui est détectée dans le passage de fluide de la soupape par rapport à une pression détectée qui est externe à la soupape.

2. Dispositif gonflable actionné par fluide implantable (100) selon la revendication 1, dans lequel l'au moins une pompe (P1, P2) inclut un dispositif à pompe(s) et soupape(s) combinées qui est positionné en ligne entre le réservoir (102) et l'élément gonflable (104), incluant :
une chambre (920) ;
un diaphragme (930) qui est positionné le long d'une partie de bord de la chambre (920) ;
un élément piézoélectrique (910) qui est monté sur le diaphragme (930) ;
une première soupape (921) qui est positionnée au niveau d'une première partie d'extrémité de la chambre (920) qui correspond à une première partie d'extrémité de l'élément piézoélectrique (910) ; et
une seconde soupape (922) qui est positionnée au niveau d'une seconde partie d'extrémité de la chambre (920) qui correspond à une seconde partie d'extrémité de l'élément piézoélectrique.

3. Dispositif gonflable actionné par fluide implantable (100) selon la revendication 2, dans lequel :
dans un premier mode dans lequel du fluide est déplacé au travers du dispositif à pompe(s) et soupape(s) combinées dans une première direction pour transférer du fluide depuis le réservoir (102) jusqu'à l'élément gonflable (104) afin de gonfler l'élément gonflable (104), un premier cycle de pompage du dispositif à pompe(s) et soupape(s) combinées inclut :
une première course d'alimentation selon laquelle du fluide est soutiré à l'intérieur de la chambre (920) au travers de la première soupape (921) tandis que la seconde soupape (922) est fermée ; et
une première course de pression selon laquelle du fluide est expulsé hors de la chambre (920) au travers de la seconde soupape (922) tandis que la première soupape (921) est fermée ; et
dans un second mode dans lequel du fluide est déplacé au travers du dispositif à pompe(s) et soupape(s) combinées dans une seconde direction pour transférer du fluide depuis l'élément gonflable (104) jusqu'au réservoir (102) afin de dégonfler l'élément gonflable (104), un second cycle de pompage du dispositif à pompe(s) et soupape(s) combinées inclut :
une seconde course d'alimentation selon laquelle du fluide est soutiré à l'intérieur de la chambre (920) au travers de la seconde soupape (922) tandis que la première soupape (921) est fermée ; et
une seconde course de pression selon laquelle du fluide est expulsé hors de la chambre au travers de la première soupape (921) tandis que la seconde soupape (922) est fermée.

4. Dispositif gonflable actionné par fluide implantable (100) selon la revendication 3, dans lequel :
la première course d'alimentation et la première course de pression sont mises en œuvre en alternance et de façon répétée jusqu'à ce qu'une pression de gonflage de l'élément gonflable (104) soit atteinte sur la base d'une pression qui est détectée par l'au moins un dispositif de détection (S1, S2) ; et
la seconde course d'alimentation et la seconde course de pression sont mises en œuvre en alternance et de façon répétée jusqu'à ce qu'une pression de dégonflage soit atteinte sur la base d'une pression qui est détectée par l'au moins un dispositif de détection (S1, S2).

5. Dispositif gonflable actionné par fluide implantable (100) selon l'une quelconque des revendications 1 à 4, dans lequel la tension variable qui est appliquée sur l'élément piézoélectrique (410) pour maintenir l'état défini du dispositif gonflable actionné par fluide (100) est basée sur la pression qui est détectée dans le passage de fluide de la soupape par rapport à une pression atmosphérique qui est détectée par le système de commande électronique (108).

6. Dispositif gonflable actionné par fluide implantable (100) selon l'une quelconque des revendications 1 à 5, dans lequel la tension variable qui est appliquée sur l'élément piézoélectrique (410) règle une position de l'élément piézoélectrique (410) et du diaphragme (430) de manière à régler au moins un paramètre parmi une pression de fluide et un débit d'écoulement de fluide afin d'effectuer un réglage vis-à-vis de conditions atmosphériques et d'assurer une correspondance vis-à-vis de l'état défini du dispositif gonflable commandé par fluide.

7. Dispositif gonflable actionné par fluide implantable (100) selon l'une quelconque des revendications 1 à 6, dans lequel la tension qui est appliquée sur l'élément piézoélectrique (410) est sélectionnée à partir d'une courbe d'étalonnage qui est associée à la soupape piézoélectrique et qui peut être accédée dans une mémoire du système de commande électronique (108).

8. Dispositif gonflable actionné par fluide implantable (100) selon l'une quelconque des revendications 1 à 7, dans lequel le dispositif à pompe(s) et soupape(s) inclut une soupape piézoélectrique normalement ouverte (400) qui est configurée pour effectuer une transition depuis un état normalement ouvert jusqu'à un état fermé en réponse à une application de tension sur l'élément piézoélectrique (410), et pour retourner dans l'état normalement ouvert en réponse à la libération de la tension.

9. Dispositif gonflable actionné par fluide implantable (100) selon la revendication 8, dans lequel la soupape piézoélectrique normalement ouverte (400) est configurée pour rester dans l'état fermé pendant une période de temps après la libération de la tension et pour effectuer une transition jusqu'à l'état normalement ouvert en réponse à la dissipation de la polarisation électrique qui est accumulée dans l'élément piézoélectrique (410).

10. Dispositif gonflable actionné par fluide implantable (100) selon la revendication 8 ou 9, dans lequel la soupape piézoélectrique normalement ouverte (400) comprend en outre une résistance (490) connectée électriquement à l'élément piézoélectrique (410), et dans lequel la résistance (490) est configurée pour commander une dissipation de la polarisation électrique qui est accumulée dans l'élément piézoélectrique (410) de telle sorte que la soupape piézoélectrique normalement ouverte (400) effectue une transition depuis l'état fermé jusqu'à l'état normalement ouvert dans une période de temps définie après la libération de tension.

11. Dispositif gonflable actionné par fluide implantable (100) selon l'une quelconque des revendications 1 à 10, dans lequel la soupape piézoélectrique est une soupape piézoélectrique normalement fermée (800) qui est configurée pour effectuer une transition depuis un état normalement fermé jusqu'à un état ouvert en réponse à une application de tension sur l'élément piézoélectrique (810), et pour retourner dans l'état normalement fermé en réponse à la libération de la tension, la soupape piézoélectrique normalement fermée (800) incluant :
un plongeur (870) qui est positionné de façon mobile à l'intérieur du passage de fluide de la soupape piézoélectrique normalement fermée (800), dans lequel le plongeur (870) est rendu étanche vis-à-vis de la base de soupape dans l'état normalement fermé de manière à restreindre l'écoulement au travers du passage de fluide et est espacé de la base de soupape dans l'état ouvert de manière à ouvrir le passage de fluide.

12. Dispositif gonflable actionné par fluide implantable (100) selon la revendication 11, dans lequel, dans l'état normalement fermé de la soupape piézoélectrique normalement fermée (800), une contre-pression qui est appliquée sur le plongeur (870) par l'intermédiaire de l'au moins une sortie maintient la position rendue étanche du plongeur (870) contre la base de soupape en réponse à une surpression de fluide au niveau de l'au moins une entrée (842).

13. Dispositif gonflable actionné par fluide implantable (100) selon l'une quelconque des revendications 1 à 12, dans lequel le système de commande électronique (108) inclut une carte de circuit imprimé qui inclut une mémoire configurée pour stocker au moins un algorithme de commande, un module de communication configuré pour communiquer avec un ou plusieurs dispositifs externes et un processeur configuré pour :
recevoir des mesures de niveau de pression en provenance de l'au moins un dispositif de détection ;
appliquer l'au moins un algorithme de commande sur la base des mesures de niveau de pression reçues ; et
commander le fonctionnement de l'au moins une soupape et de l'au moins une pompe en fonction de l'au moins un algorithme de commande appliqué.

14. Dispositif gonflable actionné par fluide implantable (100) selon l'une quelconque des revendications 1 à 13, dans lequel le dispositif gonflable actionné par fluide implantable (100) est un sphincter urinaire artificiel (100A) ou une prothèse pénienne gonflable (100B).
